# EUROPÄISCHE PATENTSCHRIFT

(11) **EP 1 534 193 B1**
(45) Veröffentlichungstag und Bekanntmachung des Hinweises auf die Patenterteilung: **05.07.2006**
(21) Anmeldenummer: 02754100.2
(22) Anmeldetag: 02.09.2002
(51) Int. Cl.: A61F 2/44

(54) **ZWISCHENWIRBELIMPLANTAT MIT DREITEILIGEM GELENK**
INTERVERTEBRAL IMPLANT COMPRISING A THREE-PART ARTICULATION
IMPLANT INTERVERTEBRAL PRESENTANT UNE ARTICULATION EN TROIS PARTIES

(43) Veröffentlichungstag der Anmeldung: 01.06.2005
(73) Patentinhaber: Synthes GmbH, 4436 Oberdorf (CH)
(72) Erfinder: BAUMGARTNER, Daniel, CH-4702 Oensingen (CH); BURRI, Adrian, CH-3900 Brig (CH); MATHIEU, Claude, 8002 Zurich (CH)
(74) Vertreter: Lusuardi, Werther
(86) Internationale Anmeldenummer: PCT/CH2002/000476
(87) Internationale Veröffentlichungsnummer: WO 2004/019828

(56) Entgegenhaltungen:
- EP-A- 0 176 728
- WO-A-99/53871
- DE-A- 3 023 353

## Beschreibung

Die Erfindung bezieht sich auf ein Zwischenwirbelimplantat gemäss dem Oberbegriff des Patentanspruchs 1.

Nach Entfernung einer beschädigten, natürlichen Bandscheibe oder eines beschädigten Nukleus einer Bandscheibe werden Implantate oder Prothesen in den Zwischenwirbelraum zweier benachbarter Wirbelkörper eingebracht. Dabei besteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglickeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die Verdrehung der benachbarten Wirbelkörper relativ zueinander soll ebenfalls innerhalb der natürlichen Grenzen ermöglicht werden.

Eine gattungsgemässe Bandscheibenendoprothese ist aus der EP 0 176 728 BÜTTNER bekannt. Diese bekannte Bandscheibenendoprothese besteht im wesentlichen aus zwei symmetrischen, konkaven Abschlussplatten, deren aussenstehende Oberflächen je an einer der Endplatten der benachbarten Wirbelkörper zur Anlage bringbar sind, und einem konvexen Distanzstück, welches zwischen den einander gegenüberliegend angeordneten, konkaven Seiten der Abschlussplatten positioniert ist. Nachteilig an dieser bekannten Bandscheibenendoprothese ist, dass
- die Drehachsen für die Flexion/Extension sowie für die laterale Beugung der benachbarten Wirbelkörper durch die gelenkartige Verbindung zwischen den Abschlussplatten nur innerhalb eines gewissen Bereiches festgelegt sind und mit den natürlichen physiologischen Verhältnissen nicht übereinstimmen;
- der Bewegungsspielraum lateral und ventral/dorsal gleich ist, da die Prothese mit dem Pe-Inlay eine rotationssymmetrische Form aufweist. Die natürlichen Durchschnittswerte für den Bewegungsspielraum, d.h. für die Rotation der Abschlussplatten um die quer zur Wirbelsäulenlängsachse verlaufenden Drehachsen liegen für die Flexion bei 10°, der Extension bei 5° und lateral bei 7°;
- die Prothese keine bewegungseinschränkenden Mittel für eine relative Rotation der Abschlussplatten um eine zur Wirbelsäulenlängsachse koaxiale oder parallele Drehachse (Torsionsbewegung der Wirbelkörper) umfasst; und
- die Prothese nur eine geringe Dämpfung bei einer Schockeinwirkung gewährleistet.

Hier will die Erfindung Abhilfe schaffen. Der Erfindung liegt die Aufgabe zugrunde, ein Zwischenwirbelimplantat zu schaffen, welches je eine definierte Drehachse für die laterale Bewegung der Wirbelsäule und für Flexion und Extension der benachbarten Wirbelkörper umfasst, wobei die Drehachsen sich kreuzen und unter verschiedenen Winkeln zur Zentralachse verlaufen.

Die Erfindung löst die gestellte Aufgabe mit einem Zwischenwirbelimplantat, insbesondere einem Zwischenwirbelimplantat, welches die Merkmale des Anspruchs 1 aufweist.

Das erfindungsgemässe Zwischenwirbelimplantat umfasst eine zur Wirbelsäulenlängsachse im wesentlichen parallele oder koaxiale Zentralachse, ein unteres und ein oberes Endstück, welche je eine quer zur Zentralachse gerichtete aussenstehende Oberfläche und einander gegenüberliegend je eine konkave Innenfläche aufweisen, sowie einen zwischen den Endstücken an den konkaven Innenflächen gleitbar positionierten Gelenkkörper. Die aussenstehenden Oberflächen der Endstücke sind je an eine der beiden Endflächen zweier benachbarter Wirbelkörper zur Anlage bringbar. Die am oberen Endstück angeordnete, erste konkave Innenfläche ist als Teilfläche einer bezüglich einer ersten, quer zur Zentralachse verlaufenden Drehachse rotationssymmetrischen Mantelfläche ausgebildet. Die am unteren Endstück angeordnete, zweite konkave Innenfläche ist als Teilfläche einer bezüglich einer zweiten, quer zur Zentralachse verlaufenden Drehachse rotationssymmetrischen Kegelmantelfläche ausgebildet, so dass die zweite Drehachse mit der Längsachse des Kegels zusammenfällt.

Im folgenden werden das untere Endstück als feststehend und der Gelenkkörper sowie das obere Endstück als bewegbar angenommen. Damit ist die zweite Drehachse, welche durch die zweite konkave, bezüglich der zweiten Drehachse rotationssymmetrische Innenfläche bestimmt wird und somit relativ zum unteren Endstück fest ist, ebenfalls feststehend, während die erste Drehachse, welche durch die erste konkave, bezüglich der ersten Drehachse rotationssymmetrischen Innenfläche bestimmt wird und somit relativ zum obere Endstück fest ist, zusammen mit dem Gelenkkörper und dem oberen Endstück um die zweite Drehachse rotiert wird, wenn der Gelenkkörper zusammen mit dem oberen Endstück um die zweite Drehachse rotiert werden. Die Zentralachse wird im folgenden ebenfalls als gegenüber dem unteren Endstück feststehend betrachtet.

In der bevorzugten Ausführungsform des erfindungsgemässen Zwischenwirbelimplantates kreuzen sich die Drehachsen. Vorzugsweise schneiden die Drehachse die Zentralachse.

Die durch die Erfindung erreichten Vorteile sind im wesentlichen darin zu sehen, dass dank des erfindungsgemässen Zwischenwirbelimplantates die beiden Drehachsen in zwei verschiedenen, quer zur Zentralachse stehenden Ebenen liegen können. Auf diese Weise lassen sich die Drehachse für laterale Biegung der Wirbelsäule und die Drehachse für Extension oder Flexion der Wirbelsäule relativ zueinander versetzt und in nicht parallelen Ebenen liegend anordnen. Durch diese Ausgestaltung lassen sich die Positionen der natürlichen Drehachsen exakt nachbilden, wodurch die Biomechanik der zwei benachbarten Wirbelkörper weitgehend an den gesunden Zustand der Wirbelsäule angenähert werden kann oder diese ganz wieder herstellbar ist. Infolge der Berücksichtigung der physiologischen Drehzentren treten somit unter Beibehaltung der Abstände zwischen Drehzentrum und Kraftansatzpunkt keine erhöhten Momente auf die Bänder, Sehnen und Muskeln auf.

In einer anderen Ausführungsform des erfindungsgemässen Zwischenwirbelimplantates sind die Gleitflächen des Gelenkkörpers nicht komplementär zu den konkaven Innenflächen der Endstücke ausgestaltet, so dass ein punktförmiger oder linienförmiger Kontakt zwischen den Gleitflächen und den konkaven Innenflächen der beiden Endstücke gebildet wird. Die Ausgestaltung mit einem linienförmigen Kontakt ist beispielsweise so ausführbar, dass die Gleitflächen am Gelenkkörper einen kleineren Krümmungsradius aufweisen als die angrenzenden konkaven Innenflächen der Endstücke. Ein punktförmiger Kontakt zwischen der ersten konkaven Innenfläche und der angrenzenden Gleitfläche am Gelenkkörper kann beispielsweise durch eine sphärische, rotationsellipsoidartige oder tonnenartige Ausgestaltung der Gleitfläche am Gelenkkörper und je nach Ausführungsform auch der ersten konkaven Innenfläche hergestellt werden.

In wiederum einer anderen Ausführungsform des erfindungsgemässen Zwischenwirbelimplantates umfasst der Gelenkkörper mindestens eine, quer zur Zentralachse angeordnete konvexe Gleitfläche, welche komplementär zur konkaven Innenfläche des angrenzenden Endstückes ausgebildet ist. Vorzugsweise sind beide Gleitflächen komplementär zu den konvexen Innenflächen der Endstücke ausgestaltet, wobei die erste Gleitfläche komplementär zur konkaven Innenfläche des oberen Endstückes und die zweite Gleitfläche komplementär zur konkaven Innenfläche des unteren Endstückes ausgebildet ist. Die erste konkave Innenfläche und die erste Gleitfläche sind hier als Teilflächen einer bezüglich einer ersten, quer zur Zentralachse stehenden Drehachse rotationssymmetrischen Mantelfläche ausgebildet und bilden die Gleitflächen eines ersten um die erste Drehachse rotierbaren Gelenkes. Die zweite konkave Innenfläche und die zweite Gleitfläche sind als Teilflächen einer Kegelmantelfläche ausgebildet, welche die Gleitflächen eines zweiten um eine zweite Drehachse rotierbaren Gelenkes bilden, wobei die zweite Drehachse der Längsachse des Kegels entspricht.

In einer weiteren Ausführungsform des erfindungsgemässen Zwischenwirbelimplantates sind die beiden Drehachsen so angeordnet, dass die zweite Drehachse in einer die Zentralachse enthaltenden Ebene, welche von der ersten Drehachse durchstossen wird, liegt. Dadurch ist der Vorteil erreichbar, dass die erste Drehachse und damit die Drehachse für die Flexion und Extension der Wirbelsäule bei nicht um die zweite Drehachse rotiertem Gelenkkörper senkrecht zur Zentralachse des Zwischenwirbelimplantates verläuft. Die zweite Drehachse dient für die laterale Beugung der Wirbelsäule und kann einen der natürlichen Bewegung angepassten Winkel α mit der Zentralachse einschliessen. Vorzugsweise beträgt dieser Winkel α zwischen 60° und 88°. Durch die Wahl des Winkels α können je nach Bandscheibenhöhe die unterschiedlichen Segmente der lumbalen Wirbelsäulen gemäss der Physiologie nachgebildet werden.

In wiederum einer weiteren Ausführungsform des erfindungsgemässen Zwischenwirbelimplantates ist die bezüglich der ersten Drehachse rotationssymmetrische Mantelfläche als Kreiszylindermantelfläche ausgestaltet. Die erste konvexe Innenfläche an einem der Endstücke sowie die erste Gleitfläche am Gelenkkörper sind somit als Teilflächen einer Kreiszylindermantelfläche ausgebildet, wodurch das erste Gelenk nur bezüglich der ersten Drehachse rotierbar ist. Dadurch wird eine spezifische Flexions/Extensionsbewegung ermöglicht. Ferner wird eine separate Betrachtung für dieses Bewegungsszenario möglich, da die Drehachse für die laterale Biegung anders liegt.

Anstelle der Ausbildung der bezüglich der ersten Drehachse rotationssymmetrischen Mantelfläche als Kreiszylindermantelfläche ist auch eine Ausgestaltung als Kegelmantelfläche möglich. Weitere Ausgestaltungsmöglichkeiten der bezüglich der ersten Drehachse rotationssymmetrischen Mantelfläche sind beispielsweise Oberflächenteile eines Rotationsellipsoides, eines Doppelkegels oder auch eines anderen beliebigen Rotationskörpers.

In einer anderen Ausführungsform des erfindungsgemässen Zwischenwirbelimplantates schneidet die erste Drehachse die Zentralachse, so dass bei einer Flexion oder Extension der Wirbelsäule das Rotationszentrum der beiden an das Zwischenwirbelimplantat angrenzenden Wirbelkörper auf der Zentralachse des Zwischenwirbelimplantates liegt. Die zweite Drehachse des zweiten Gelenkes schneidet die Zentralachse ebenfalls. Dabei weisen die beiden Drehachsen zueinander einen minimalen Abstand A auf. Vorzugsweise beträgt dieser Abstand A zwischen 0 mm und 18 mm. Dieser Abstand A resultiert aus den anatomischen Gegebenheiten der Rotationszentren, beispielsweise aus der Tatsache, dass die Drehachse für die laterale Biegung diagonal gegen dorsal abfallend in der Medianebene des menschlichen Körpers verläuft.

In wiederum einer weiteren Ausführungsform des erfindungsgemässen Zwischenwirbelimplantates sind die aussenstehenden Oberflächen der Endstücke mit einer dreidimensionalen Struktur versehen, welche beispielsweise durch eine Aufrauhung der Oberfläche erreichbar ist und das Anwachsen der angrenzenden Wirbelkörper an das Zwischenwirbelimplantat begünstigt.

In einer anderen Ausführungsform des erfindungsgemässen Zwischenwirbelimplantates ist anstelle der dreidimensionalen Strukturierung ein Gitter, vorzugsweise ein Titangitter an den aussenstehenden Oberflächen der Endstücke angebracht, wodurch wiederum das Anwachsen der Endplatten der Wirbelkörper am Zwischenwirbelimplantat begünstigt wird.

In wiederum einer anderen Ausführungsform des erfindungsgemässen Zwischenwirbelimplantates umfasst mindestens eines der Endstücke erste und zweite Anschlagmittel, wodurch die Bewegungen der Endstücke relativ zueinander begrenzt werden. Diese Anschlagmittel dienen zur Begrenzung der relativen Rotation der Endstücke um die erste Drehachse, wodurch die Flexion und Extension der benachbarten Wirbelkörper ermöglicht wird. Vorzugsweise wird die laterale Biegung der beiden an das Zwischenwirbelimplantat angrenzenden Wirbelkörper durch dritte Anschlagmittel eingeschränkt. Diese dritten Anschlagmittel begrenzen die relative Rotation der Endstücke um die zweite Drehachse. Die Anschlagmittel sind derart ausgestaltet, dass eine maximale Flexion der benachbarten Wirbelkörper zwischen 5° und 15°, eine maximale Extension zwischen 2° und 15° und eine maximale laterale Biegung zwischen ± 5° und ± 10° zugelassen wird.

In einer Ausführungsform des erfindungsgemässen Zwischenwirbelimplantates ist eines der Endstücke dreiteilig ausgestaltet. Dieses Endstück umfasst eine aussenstehende Deckplatte, eine die konkave Innenfläche umfassende Gelenkschale und dazwischen ein quer zur Zentralachse angeordnetes, elastisch deformierbares Zwischenteil. Durch das Einfügen dieses elastisch deformierbaren Zwischenteiles ist der Vorteil erreichbar, dass einerseits eine Kompressionsbewegung der beiden benachbarten Wirbelkörper gedämpft wird und andererseits Scherungs- und Torsionsbewegungen zwischen den beiden Endstücken des Zwischenwirbelimplantates möglich sind.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Ausführungsform des erfindungsgemässen Zwischenwirbelimplantates;
Fig. 2 eine perspektivische Darstellung des Gelenkkörpers mit den die Gleitflächen enthaltenden, zu den Drehachsen rotationssymmetrischen Mantelflächen der in Fig. 1 dargestellten Ausführungsform des erfindungsgemässen Zwischenwirbelimplantates;
Fig. 3 eine Ansicht von lateral auf eine Ausführungsform des erfindungsgemässen Zwischenwirbelimplantates;
Fig. 4 eine Ansicht von posterior auf die in Fig. 3 dargestellte Ausführungsform des erfindungsgemässen Zwischenwirbelimplantates; und
Fig. 5 eine Explosionsdarstellung einer anderen Ausführungsform des erfindungsgemässen Zwischenwirbelimplantates.

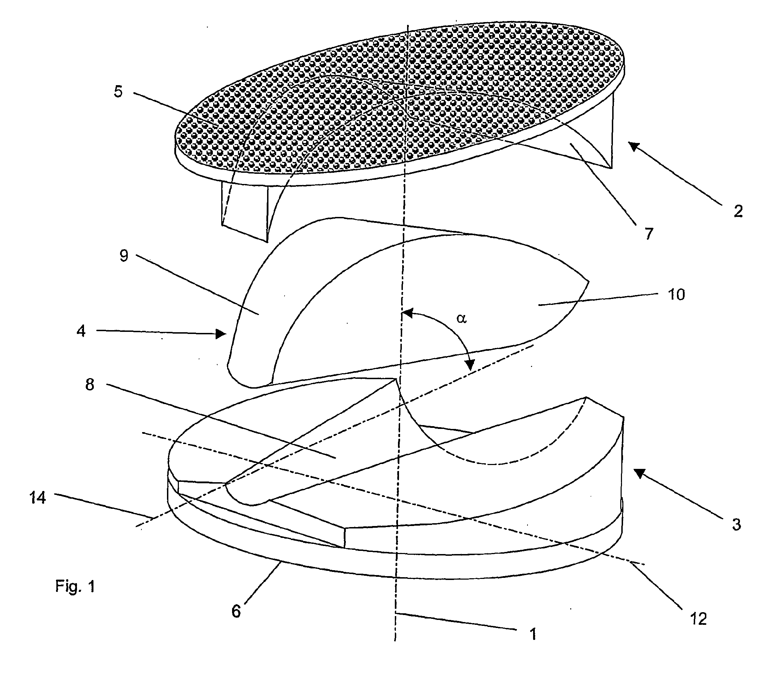

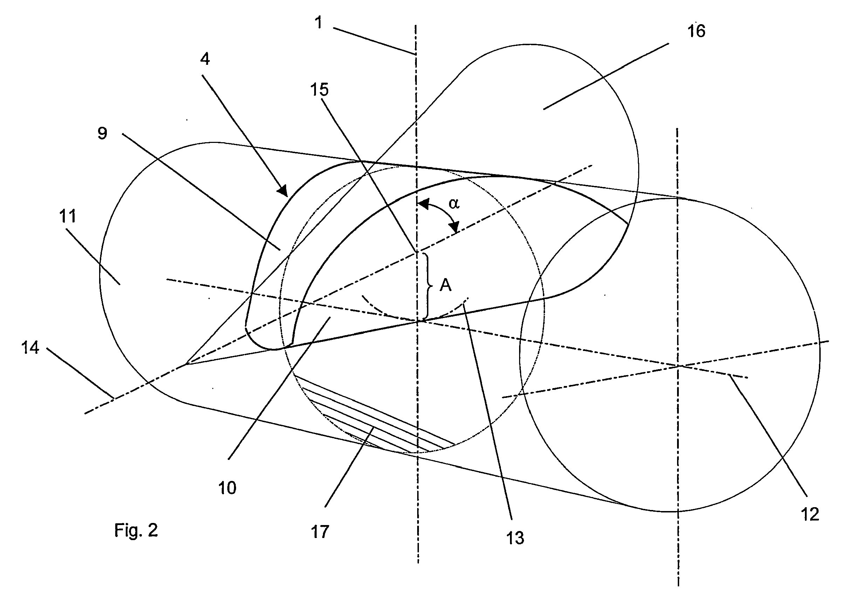

In Fig. 1 ist eine Ausführungsform des erfindungsgemässen Zwischenwirbelimplantates dargestellt, welche ein oberes und ein unteres die Zentralachse 1 schneidendes Endstück 2;3 und einen ebenfalls die Zentralachse 1 schneidenden, zwischen den Endstücken 2;3 positionierten Gelenkkörper 4 umfasst. Die beiden Endstücke 2;3 weisen je eine aussenstehende, an die Endplatten der angrenzenden Wirbelkörper angepasste und quer zur Zentralachse 1 stehende Oberfläche 5;6 auf, welche je an einer der einander gegenüberliegenden Endflächen zweier benachbarter Wirbelkörper zur Anlage bringbar sind. Einander gegenüberliegend umfassen die beiden Endstücke 2;3 je eine konkave Innenfläche 7;8, während der Gelenkkörper 4 zwei konvexe Gleitflächen 9;10 aufweist, wobei eine der konvexen Gleitflächen 9 komplementär zur konkaven Innenfläche 7 des oberen Endstückes 2 und die andere der konvexen Gleitflächen 10 komplementär zur konkaven Innenfläche 8 des unteren Endstückes 3 ausgebildet ist. Die erste konkave Innenfläche 7 sowie die dazu komplementäre, erste Gleitfläche 9 bilden die Gleitflächen eines ersten um die erste Drehachse 12 rotierbaren Gelenkes zwischen Gelenkkörper 4 und dem oberen Endstück 2. Falls sich der Gelenkkörper 4 in seiner Ausgangslage befindet, d.h. nicht um die zweite Drehachse 14 rotiert wurde, steht die erste Drehachse 12 senkrecht zur Zentralachse 1. Eine Rotation des oberen Endstückes 2 um die erste Drehachse 12 bewirkt eine Flexions- oder Extensionsbewegung der an die Endstücke 2;3 angrenzenden Wirbelkörper. Die erste konkave Innenfläche 7 am oberen Endstück 2 sowie die dazu komplementäre, erste Gleitfläche 9 am Gelenkkörper 4 sind in der hier dargestellten Ausführungsform des erfindungsgemässen Zwischenwirbelimplantates als Teilflächen einer zur ersten Drehachse 12 rotationssymmetrischen Kreiszylindermantelfläche ausgebildet. Die zweite konkave Innenfläche 8 sowie die dazu komplementäre, zweite Gleitfläche 10 bilden die Gleitflächen eines zweiten um eine zweite Drehachse 14 rotierbaren Gelenkes zwischen dem Gelenkkörper 4 und dem unteren Endstück 3. Diese zweite Drehachse 14 schneidet die Zentralachse 1 mit einem Winkel α, schneidet jedoch die erste Drehachse 12 nicht. Wie in Fig. 2 gezeigt, liegt die zweite Drehachse 14 in einer die Zentralachse 1 enthaltenden Ebene 17, welche von der ersten Drehachse 12 durchstossen wird. Eine Rotation des Gelenkkörpers 4 zusammen mit dem oberen Endstück 2 um die zweite Drehachse 14 ermöglicht somit eine laterale Beugung der beiden an das Zwischenwirbelimplantat angrenzenden Wirbelkörper. Ferner ist aus Fig. 2 ersichtlich, dass die bezüglich der ersten Drehachse 12 rotationssymmetrische Mantelfläche 11 eine Kreiszylindermantelfläche ist, wovon die erste Gleitfläche 9 einen Ausschnitt bildet. Die zweite Gleitfläche 10 ist ein Ausschnitt aus einer zur zweiten Drehachse 14 rotationssymmetrischen Kegelmantelfläche 16. Ferner weisen die beiden Drehachsen einen Abstand A zueinander auf. Wie in Fig. 2 dargestellt, bewegt sich bei einer Rotation des Gelenkkörpers 4 um die zweite Drehachse die erste Drehachse 12 auf einem zum Schnittpunkt 15 zwischen der Zentralachse 1 und der zweiten Drehachse 14 konzentrischen Bogen 13 mit dem Radius A.

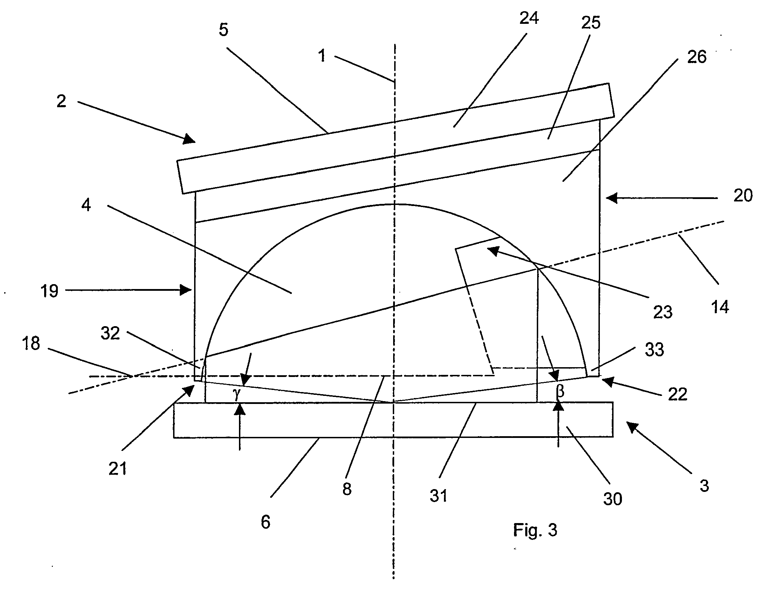

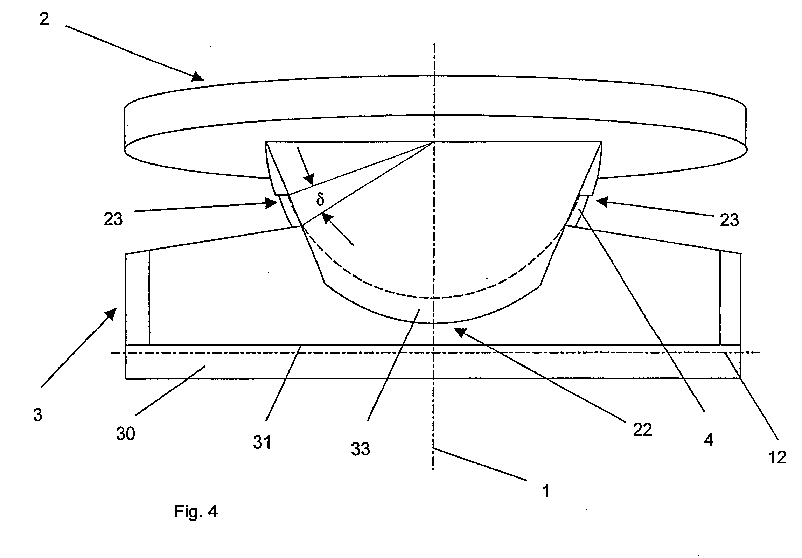

In Fig. 3 ist eine Ausführungsform des erfindungsgemässen Zwischenwirbelimplantates dargestellt, welche sich von der in Fig. 1 dargestellten Ausführungsform darin unterscheidet, dass das obere Endstück 2 dreiteilig ausgebildet ist. Das obere Endstück 2 umfasst axial aussenstehend eine die obere aussenstehende Oberfläche 5 beinhaltende Deckplatte 24, gegen den Gelenkkörper 4 gerichtet eine Gelenkschale 26 und zwischen Gelenkschale 26 und Deckplatte 24 ein elastisch deformierbares Zwischenteil 25. Ferner weist das Zwischenwirbelimplantat eine gegen die Kegelspitze 18 gerichtete Vorderseite 19 und gegenüberliegend eine Hinterseite 20 auf. Das Zwischenwirbelimplantat ist so ausgestaltet, dass die Vorderseite 19 nach der Implantation in den Zwischenwirbelraum posterior positioniert ist. Damit verläuft die zweite Drehachse 14 von anterior nach posterior. Die Rotation des oberen Endstückes 2 um die senkrecht zur Bildebende stehende erste Drehachse 12 (Fig. 1) wird durch die beiden Anschlagmittel 21; 22 begrenzt. Dabei sind die ersten Anschlagmittel 21, welche zu einer Begrenzung einer die Vorderseite 19 parallel zur Zentralachse 1 verkürzenden Rotation der beiden Endstücke 2;3 relativ zueinander dienen und damit die Extensionsbewegung der beiden angrenzenden Wirbelkörper einschränken, auf der Vorderseite 19 am oberen Endstück 2 angebracht, während die zweiten Anschlagmittel 22, welche zu einer Begrenzung einer die Hinterseite 20 parallel zur Zentralachse 1 verkürzenden Rotation der beiden Endstücke 2;3 relativ zueinander dienen und die Flexionsbewegung der beiden angrenzenden Wirbelkörper einschränken. Die ersten und zweiten Anschlagmittel 21;22 werden durch die auf der Vorderseite 19 respektive auf der Hinterseite 20 liegenden unteren Enden 32;33 des oberen Endstückes 2 gebildet, welche auf der gegen den Gelenkkörper 4 gerichteten, inneren Oberfläche 31 der Grundplatte 30 des unteren Endstückes 3 nach Erreichen des maximal zulässigen Drehwinkels anliegen. Wie in Fig. 4 gezeigt ist das untere Ende 33 auf der Hinterseite 20 (Fig. 3) abgerundet, wobei das Zentrum dieser Abrundung im Schnittpunkt der zweiten Drehachse 14 (Fig. 3) mit der Hinterseite 20 des Zwischenwirbelimplantates zusammenfällt, so dass die ersten Anschlagmittel 22 bei um die zweite Drehachse 14 (Fig. 3) gedrehten, oberen Endstück 2 unter demselben Drehwinkel um die erste Drehachse 12 an der inneren Oberfläche 31 der Grundplatte 30 des unteren Endstückes 3 zur Anlage kommen. Ferner ist am Gelenkkörper 4 die zweite Gleitfläche 10 (Fig. 1) an der Hinterseite 20 des Zwischenwirbelimplantates auf einem Teil ihrer Peripherie und auf einem Teil ihrer Länge abgesetzt. Analog dazu ist die zweiten konkave Innenfläche 8 abgesetzt. Die durch diesen Absatz ausgestalteten dritten Anschlagmittel 23 begrenzen den Drehwinkel des oberen Endstückes 2 um die zweite Drehachse 14, so dass dadurch die laterale Biegung der beiden an das Zwischenwirbelimplantat angrenzenden Wirbelkörper eingeschränkt wird. Die Anschlagmittel 21;22;23 sind derart ausgestaltet, dass eine Flexion der benachbarten Wirbelkörper um einen Winkel β von 10°, eine Extension um einen Winkel γ von 5° und eine laterale Biegung um einen Winkel δ von ± 7° zugelassen wird.

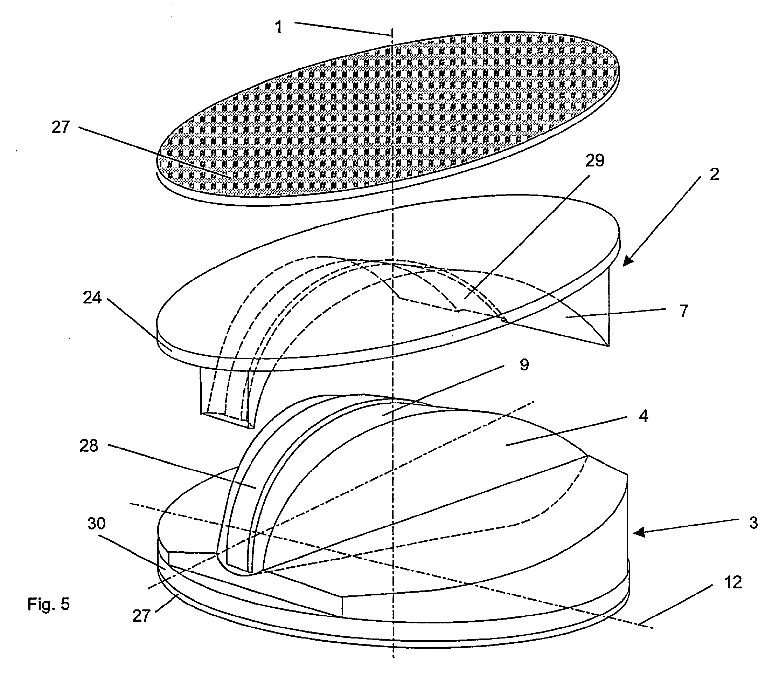

Die in Fig. 5 dargestellte Ausführungsform des erfindungsgemässen Zwischenwirbelimplantates unterscheidet sich von den in den Fig. 1, 3 oder 4 dargestellten Ausführungsformen nur darin, dass aussen an der Deckplatte 24 sowie an der Grundplatte 30 Gitternetze 27 angebracht sind und dass die erste konvexe Gleitfläche 9 eine zur ersten Drehachse 12 konzentrische Erhebung 28 und die erste konkave Innenfläche 7 eine Vertiefung 29 umfasst, wobei die Erhebung 28 bei einer Rotation des oberen Endstückes 2 relativ zum Gelenkkörper 4 in der Vertiefung 29 tangential verschoben wird. Die Erhebung 28 erstreckt sich parallel zur ersten Drehachse 12 nur über einen Teil der ersten Gleitfläche 9 während sich die periphere Ausdehnung der Erhebung 28 über die gesamte erste Gleitfläche 9 erstreckt. Die Vertiefung 29 in der ersten konkaven Innenfläche 29 ist zur Erhebung komplementär ausgestaltet. In anderen Ausführungsformen des erfindungsgemässen Zwischenwirbelwirbelimplantates sind auch Erhebungen 28 möglich, welche eine periphere Ausdehnung auf nur einem Teil der ersten Gleitfläche 9 aufweisen. Die Vorteile dieser in die Vertiefung 29 eingreifenden Erhebung 28 liegen darin, dass einerseits eine laterale Stabilisation zwischen dem ersten Endstück 2 und dem Gelenkkörper 4 erreichbar ist, eine Scherbewegung der angrenzenden Wirbelkörper parallel zur ersten Drehachse 12 verhindert wird, und andererseits eine Zentrierung des Gelenkkörpers 4 innerhalb des Zwischenwirbelimplantates erreichbar ist, so dass sich der Gelenkkörper 4 nicht parallel zur ersten Drehachse 12 gegenüber dem ersten Endstück 2 verschieben kann. Die Gitternetze 27 sind vorzugsweise als Titannetze gefertigt und können auch gekrümmt ausgestaltet sein, wodurch ein optimales Anwachsen der angrenzenden Wirbelkörper an das Zwischenwirbelimplantat ermöglicht wird.

## Patentansprüche

1. Zwischenwirbelimplantat mit einer zur Wirbelsäulenlängsachse im wesentlichen parallelen oder koaxialen Zentralachse (1) umfassend
A) ein oberes und ein unteres Endstück (2;3) mit je einer quer zur Zentralachse (1) angeordneten, aussenstehenden Oberfläche (5;6) und einander gegenüberliegend je einer konkaven Innenfläche (7;8); und
B) einen zwischen den Endstücken (2;3) positionierten, konvexen Gelenkkörper (4), welcher an den konkaven Innenflächen (7;8) der beiden Endstücke (2;3) gleitbar anliegt, wobei
C) die erste konkave Innenfläche (7) eine Teilfläche einer bezüglich einer ersten, quer zur Zentralachse (1) stehenden Drehachse (12) rotationssymmetrischen Mantelfläche (11) ist, **dadurch gekennzeichnet, daß**
D) die zweite konkave Innenfläche (8) eine Teilfläche einer bezüglich einer zweiten, quer zur Zentralachse (1) stehenden Drehachse (14) rotationssymmetrischen Kegelmantelfläche (16) ist.

2. Zwischenwirbelimplantat nach Anspruch 1, **dadurch gekennzeichnet, dass** sich die erste Drehachse (12) und die zweite Drehachse (14) kreuzen.

3. Zwischenwirbelimplantat nach Anspruch 1 oder 2, **dadurch gekennzeichnet, dass** der Gelenkkörper (4) mindestens eine die Zentralachse (1) schneidende, konvexe Gleitfläche (9;10) umfasst.

4. Zwischenwirbelimplantat nach Anspruch 3, **dadurch gekennzeichnet, dass** die Krümmungsradien der ersten konkaven Innenfläche (7) und der anliegenden Gleitfläche (9) am konvexen Gelenkkörper (4) verschieden sind und dass die Gleitfläche (7) sphärisch, ellipsoidartig oder tonnenartig ausgestaltet ist, so dass zwischen dem konvexen Gelenkkörper (4) und der ersten konkaven Innenflächen (7) eine Punktauflage herstellbar ist.

5. Zwischenwirbelimplantat nach Anspruch 3, **dadurch gekennzeichnet, dass** die Krümmungsradien mindestens einer der Gleitflächen (9;10) am konvexen Gelenkkörper (4) und mindestens einer der konkaven Innenflächen (7;8) der beiden Endstücke (2;3) verschieden sind, so dass zwischen dem konvexen Gelenkkörper (4) und einer oder beiden konkaven Innenflächen (7;8) eine Linienauflage herstellbar ist.

6. Zwischenwirbelimplantat nach Anspruch 3 , **dadurch gekennzeichnet, dass** die erste Gleitfläche (9) des Gelenkkörpers (4) komplementär zur konkaven Innenfläche (7) des oberen Endstückes (2) ausgestaltet ist und die konkave Innenfläche (7) zusammen mit der ersten Gleitfläche (9) die Gleitflächen eines ersten um die erste Drehachse (12) rotierbaren Gelenkes bilden.

7. Zwischenwirbelimplantat nach Anspruch 3 oder 6, **dadurch gekennzeichnet, dass** die zweite Gleitfläche (10) des Gelenkkörpers (4) komplementär zur konkaven Innenfläche (8) des unteren Endstückes (3) ausgestaltet ist und die konkave Innenfläche (8) zusammen mit der zweiten Gleitfläche (10) die Gleitflächen eines zweiten um die zweite Drehachse (14) rotierbaren Gelenkes bilden.

8. Zwischenwirbelimplantat nach einem der Ansprüche 1 bis 7, **dadurch gekennzeichnet, dass** die zweite Drehachse (14) die Zentralachse (1) unter einem Winkel α schneidet und der Winkel α zwischen 60° und 88° beträgt.

9. Zwischenwirbelimplantat nach einem der Ansprüche 1 bis 8, **dadurch gekennzeichnet, dass** die rotationssymmetrische Mantelfläche (11) eine Kreiszylindermantelfläche ist.

10. Zwischenwirbelimplantat nach einem der Ansprüche 1 bis 8, **dadurch gekennzeichnet, dass** die rotationssymmetrische Mantelfläche (11) eine zweite Kegelmantelfläche ist.

11. Zwischenwirbelimplantat nach einem der Ansprüche 1 bis 10, **dadurch gekennzeichnet, dass** die erste Drehachse (12) und die zweite Drehachse (14) einen minimalen Abstand A zueinander aufweisen.

12. Zwischenwirbelimplantat nach Anspruch 11, **dadurch gekennzeichnet, dass** der Abstand A zwischen 0 mm und 18 mm beträgt.

13. Zwischenwirbelimplantat nach einem der Ansprüche 1 bis 12, **dadurch gekennzeichnet, dass** die aussenstehenden Oberflächen (5;6) eine dreidimensionale Strukturierung aufweisen.

14. Zwischenwirbelimplantat nach einem der Ansprüche 1 bis 13, **dadurch gekennzeichnet, dass** die aussenstehenden Oberflächen (5;6) Titangitter sind, welche mit den Endstücken (2;3) verbindbar sind.

15. Zwischenwirbelimplantat nach einem der Ansprüche 1 bis 14, **dadurch gekennzeichnet, dass**
a) die Kegelmantelfläche (16) eine auf der zweiten Drehachse (14) liegende Kegelspitze (18) umfasst;
b) das Zwischenwirbelimplantat eine gegen die Kegelspitze (18) gerichtete Vorderseite (19) und entgegengesetzt eine Hinterseite (20) aufweist;
c) mindestens eines der Endstücke (2;3) erste Anschlagmittel (21) umfasst, welche eine die Vorderseite (19) des Zwischenwirbelimplantates parallel zur Zentralachse (1) verkürzende Rotation um die erste Drehachse (12) bei einem Drehwinkel β zwischen 5° und 15° begrenzt; und
d) mindestens eines der Endstücke (2;3) zweite Anschlagmittel (22) umfasst, welche eine die Hinterseite (20) des Zwischenwirbelimplantates parallel zur Zentralachse (1) verkürzende Rotation um die erste Drehachse (12) bei einem Drehwinkel γ zwischen 2° und 15° begrenzt.

16. Zwischenwirbelimplantat nach einem der Ansprüche 1 bis 15, **dadurch gekennzeichnet, dass** es dritte Anschlagmittel (23) umfasst, welche die Rotation um die zweite Drehachse (14) bei einem maximalen Drehwinkel δ von zwischen ± 5° und ± 10° begrenzen.

17. Zwischenwirbelimplantat nach einem der Ansprüche 1 bis 16, **dadurch gekennzeichnet, dass** mindestens eines der Endstücke (2;3) dreiteilig ausgestaltet ist und eine aussenstehende Deckplatte (24), eine die konkave Innenfläche (7;8) umfassende Gelenkschale (26) und dazwischen ein elastisch deformierbares Zwischenteil (25) umfasst.

## Claims

1. An intervertebral implant having a central axis (1) substantially parallel to or coaxial with the spinal column's axis, comprising
(A) an upper and a lower terminal part (2; 3) each fitted with an outermost surface (5; 6) configured transversely to the central axis (1) and each with a concave inner surface (7; 8) which are mutually opposite; and
(B) a convex joint element (4) configured between the terminal parts (2; 3) and resting in sliding manner against the concave inner surfaces (7; 8) of the two terminal parts (2; 3), whereby
(C) the first concave inner surface (7) is a partial surface of a first external surface which is rotationally symmetrical about an axis of rotation (12) transverse to the central axis (1), and
**characterized in that**
(D) the second concave surface (8) is a partial surface of a second rotationally symmetrical conical external surface (16) having an axis of rotation (14) transverse to the central axis (1).

2. Intervertebral implant as claimed in claim 1, **characterized in that** the first axis of rotation (12) and the second axis of rotation (14) cross each other.

3. Intervertebral implant as claimed in either of claims 1 and 2, **characterized in that** the joint element (4) comprises at least one convex slide surface (9; 10) intersecting the central axis (1).

4. Intervertebral implant as claimed in claim 3, **characterized in that** the radii of curvature of the first concave inner surface (7) and of the slide surface (9) resting against the convex joint element (4) are different and **in that** the slide surface (7) is spherical, ellipsoidal or barrel-like whereby a point-like rest is made possible between the convex joint element (4) and the first concave inner surfaces (7).

5. Intervertebral implant as claimed in claim 3, **characterized in that** the radii of curvature of at least one of the slide surfaces (9; 10) at the convex joint element (4) and of at least one of the concave inner surfaces (7; 8) of the two terminal parts (2; 3) are different whereby linear rest may be implemented between the convex joint element (4) and one or both concave inner surfaces (7; 8).

6. Intervertebral implant as claimed in claim 3, **characterized in that** the first slide surface (9) of the joint element (4) is complementary to the concave inner surface (7) of the upper terminal part (2) and that the concave inner surface (7) together with the first slide surface (9) constitute the slide surfaces of a first joint rotatable about the first axis of rotation (12).

7. Intervertebral implant as claimed in either of claims 3 and 6, **characterized in that** the second slide surface (10) of the joint element (4) is complementary to the concave inner surface (8) of the lower terminal part (3) and **in that** the concave inner surface (8) together with the second slide surface (10) constitute the slide surfaces of a second joint rotatable about the second axis of rotation (14).

8. Intervertebral implant as claimed in one of claims 1 through 7, **characterized in that** the second axis of rotation (14) intersects the central axis (1) at an angle α between 60 and 88°.

9. Intervertebral implant as claimed in one of claims 1 through 8, **characterized in that** the rotationally symmetrical external surface (11) is a circularly cylindrical external surface.

10. Intervertebral implant as claimed in one of claims 1 through 8, **characterized in that** the rotationally symmetrical external surface (11) is a second conical external surface.

11. Intervertebral implant as claimed in one of claims 1 through 10, **characterized in that** the first axis of rotation (12) and the second axis of rotation (14) are apart by a minimum distance A.

12. Intervertebral implant as claimed in claim 11, **characterized in that** the distance A is between 0 and 18 mm.

13. Intervertebral implant as claimed in one of claims 1 through 12, **characterized in that** the outermost surfaces (5; 6) exhibit a three-dimensional structure.

14. Intervertebral implant as claimed in one of claims 1 through 13, **characterized in that** the outermost surfaces (5; 6) are titanium grids that can be connected to the terminal parts (2; 3).

15. Intervertebral implant as claimed in one of claims 1 through 14, **characterized in that**
(a) the conical external surface (16) comprises a cone tip (18) situated on the second axis of rotation (14);
(b) the intervertebral implant comprises a front side (19) pointing at the cone tip (18) and opposite a rear side (20);
(c) at least one of the terminal parts (2; 3) comprises a first rotation-restricting stop (21) shortening the front side (19) of the intervertebral implant parallel to the central axis (1) about the first axis of rotation (12) at an angle of rotation β between 5 and 15°; and
(d) at least one of the terminal parts (2; 3) includes a rotation-restricting stop (22) shortening the rear side (20) of the intervertebral implant parallel to the central axis (1) about the first axis of rotation at an angle of rotation γ between 2 and 15°.

16. Intervertebral implant as claimed in one of claims 1 through 15, **characterized in that** it includes a third stop (23) restricting the rotation about the second axis of rotation (14) at a maximum angle of rotation δ between ± 5° and ± 10°.

17. Intervertebral implant as claimed in one of claims 1 through 16, **characterized in that** at least one of the terminal parts (2; 3) is a three-element part and comprises an outermost cover plate (24), a joint pan (26) enclosing the concave inner surface (7; 8) and in-between an elastically deforming spacer (25).

## Revendications

1. Implant intervertébral ayant un axe central (1) essentiellement parallèle ou coaxial à l'axe longitudinal de la colonne vertébrale, comprenant
A) une pièce d'extrémité supérieure et inférieure (2 ; 3) ayant chacune une surface (5 ; 6) tournée vers l'extérieur, disposée transversalement à l'axe central (1) et une surface interne concave (7 ; 8) face à face ; et
B) un corps d'articulation convexe (4) positionné entre les pièces d'extrémité (2 ; 3), lequel repose de manière à pouvoir glisser sur les surfaces internes concaves (7 ; 8) des deux pièces d'extrémité (2 ; 3),
dans lequel
C) la première surface interne concave (7) est une surface partielle d'une surface latérale (11) avec une symétrie de rotation par rapport à un premier axe de rotation (12) situé transversalement à l'axe central (1) ;
**caractérisé en ce que**
D) la deuxième surface interne concave (8) est une surface partielle d'une surface latérale conique (16) avec une symétrie de rotation par rapport à un deuxième axe de rotation (14) situé transversalement à l'axe central (1).

2. Implant intervertébral selon la revendication 1, **caractérisé en ce que** le premier axe de rotation (12) et le deuxième axe de rotation (14) se croisent.

3. Implant intervertébral selon la revendication 1 ou 2, **caractérisé en ce que** le corps d'articulation (4) comprend au moins une surface de glissement (9 ; 10) convexe coupant l'axe central (1).

4. Implant intervertébral selon la revendication 3, **caractérisé en ce que** les rayons de courbure de la première surface interne concave (7) et de la surface de glissement adjacente (9) sur le corps d'articulation convexe (4) sont différents, et **en ce que** la surface de glissement (7) est réalisée sous une forme sphérique, ellipsoïdale ou de tonneau, de sorte qu'un appui ponctuel est réalisable entre le corps d'articulation convexe (4) et la première surface interne concave (7).

5. Implant intervertébral selon la revendication 3, **caractérisé en ce que** les rayons de courbure d'au moins une des surfaces de glissement (9 ; 10) sur le corps d'articulation convexe (4) et d'au moins une des surfaces internes concaves (7 ; 8) des deux pièces d'extrémité (2 ; 3) sont différents, de sorte qu'un appui linéaire est réalisable entre le corps d'articulation convexe (4) et une ou les deux surfaces internes concaves (7 ; 8).

6. Implant intervertébral selon la revendication 3, **caractérisé en ce que** la première surface de glissement (9) du corps d'articulation (4) est réalisée de manière complémentaire à la surface interne concave (7) de la pièce d'extrémité supérieure (2), et **en ce que** la surface interne concave (7) conjointement avec la première surface de glissement (9) forment les surfaces de glissement d'une première articulation pouvant tourner autour du premier axe de rotation (12).

7. Implant intervertébral selon la revendication 3 ou 6, **caractérisé en ce que** la deuxième surface de glissement (10) du corps d'articulation (4) est réalisée de manière complémentaire à la surface interne concave (8) de la pièce d'extrémité inférieure (3), et **en ce que** la surface interne concave (8) conjointement avec la deuxième surface de glissement (10) forment les surfaces de glissement d'une deuxième articulation pouvant tourner autour du deuxième axe de rotation (14).

8. Implant intervertébral selon l'une quelconque des revendications 1 à 7, **caractérisé en ce que** le deuxième axe de rotation (14) coupe l'axe central (1) selon un angle α, et l'angle α est compris entre 60° et 88°.

9. Implant intervertébral selon l'une quelconque des revendications 1 à 8, **caractérisé en ce que** la surface latérale avec symétrie de rotation (11) est une surface latérale cylindrique.

10. Implant intervertébral selon l'une quelconque des revendications 1 à 8, **caractérisé en ce que** la surface latérale avec symétrie de rotation (11) est une deuxième surface latérale conique.

11. Implant intervertébral selon l'une quelconque des revendications 1 à 10, **caractérisé en ce que** le premier axe de rotation (12) et le deuxième axe de rotation (14) présentent entre eux une distance minimale A.

12. Implant intervertébral selon la revendication 11, **caractérisé en ce que** la distance A est comprise entre 0 mm et 18 mm.

13. Implant intervertébral selon l'une quelconque des revendications 1 à 12, **caractérisé en ce que** les surfaces tournées vers l'extérieur (5 ; 6) présentent une texture tridimensionnelle.

14. Implant intervertébral selon l'une quelconque des revendications 1 à 13, **caractérisé en ce que** les surfaces tournées vers l'extérieur (5 ; 6) sont des treillis de titane, lesquels peuvent être fixés aux pièces d'extrémité (2 ; 3).

15. Implant intervertébral selon l'une quelconque des revendications 1 à 14, **caractérisé en ce que**
a) la surface latérale conique (16) comprend une pointe de cône (18) située sur le deuxième axe de rotation (14) ;
b) l'implant intervertébral présente une face avant (19) tournée vers la pointe de cône (18) et une face arrière (20) opposée ;
c) au moins une des pièces d'extrémité (2 ; 3) comprend un premier moyen de butée (21), lequel limite une rotation, réduisant la face avant (19) de l'implant intervertébral parallèlement à l'axe central (1), autour du premier axe de rotation (12) à un angle de rotation β compris entre 5° et 15°; et
d) au moins une des pièces d'extrémité (2 ; 3) comprend un deuxième moyen de butée (22), lequel limite une rotation, réduisant la face arrière (20) de l'implant intervertébral parallèlement à l'axe central (1), autour du premier axe de rotation (12) à un angle de rotation γ compris entre 2° et 15°.

16. Implant intervertébral selon l'une quelconque des revendications 1 à 15, **caractérisé en ce qu'**il comprend un troisième moyen de butée (23), lequel limite la rotation autour du deuxième axe de rotation (14) à un angle de rotation maximale δ compris entre ± 5° et ± 10°.

17. Implant intervertébral selon l'une quelconque des revendications 1 à 16, **caractérisé en ce qu'**au moins une des pièces d'extrémité (2 ; 3) est réalisée en trois parties et comprend une plaque de recouvrement tournée vers l'extérieur (24), une coque d'articulation (26) comprenant la surface interne concave (7 ; 8) et, entre elles, un élément intermédiaire (25) à déformation élastique.
